# EUROPÄISCHE PATENTSCHRIFT

(11) **EP 0 837 053 B1**
(45) Veröffentlichungstag und Bekanntmachung des Hinweises auf die Patenterteilung: **15.01.2003**
(21) Anmeldenummer: 97117688.8
(22) Anmeldetag: 13.10.1997
(51) Int. Cl.: C07C 249/02

(54) **Verfahren zur Entfernung von Wasser und Ammoniak aus Benzophenonimin-Reaktionsausträgen**
Process for the elimination of water and ammonia from benzophenone imine reaction effluents
Procédé d'élimination d'eau et d'ammoniaque d'effluents obtenus lors de la préparation de benzophénone imine

(30) Priorität: 15.10.1996 DE 19642541
(43) Veröffentlichungstag der Anmeldung: 22.04.1998
(73) Patentinhaber: BASF AKTIENGESELLSCHAFT, 67056 Ludwigshafen (DE)
(72) Erfinder: Voit, Guido, Dr., 69198 Schriesheim (DE); Dernbach, Matthias, Dr., 69214 Eppelheim (DE); Beck, Karl, 76684 Östringen (DE); Holderbaum, Martin, Dr., 55765 Birkenfeld (DE); Weyer, Hans-Jürgen, Dr., 67240 Bodenheim-Roxheim (DE)
(74) Vertreter: Isenbruck, Günter, Dr.

(56) Entgegenhaltungen:
- EP-A- 0 713 861
- US-A- 4 130 586
- GIANCARLO VERARDO ET AL.: "Ketimines from Ketones and Ammonia" SYNTHETIC COMMUNICATIONS, Bd. 18, Nr. 13, 1988, Seiten 1501-1511, XP002052833

## Beschreibung

Die vorliegende Erfindung betrifft ein Verfahren zur Entfernung von Wasser und Ammoniak aus Benzophenonimin-Reaktionsausträgen.

Verschiedene Syntheseverfahren zur Herstellung von Benzophenoniminen sind bekannt. Neben der Herstellung durch Umsetzung von Grignardreagenzien mit Nitrilen und anschließender Hydrolyse des Reaktionsproduktes oder Umsetzung von geminalen Dihalogeniden mit flüssigem Ammoniak werden vornehmlich katalytische Umsetzungen von Benzophenon mit Ammoniak zu Benzophenonimin und Wasser durchgeführt.

Als Katalysatoren kommen dabei neben organischen Säuren, Ionenaustauschern und Chloriden, wie Eisen(III)chlorid, Ammoniumchlorid, Zink(II)chlorid auch Metalloxide zum Einsatz, wie Thoriumoxid/Siliciumoxid oder Oxide von Metallen der zweiten bis fünften Periode der III. bis V. Hauptgruppe des Periodensystems der Elemente.

Die Umsetzung erfolgt dabei vorzugsweise mit flüssigem Ammoniak, wobei hohe Ausbeuten erhalten werden.

Bei der Umsetzung von Benzophenon und Ammoniak zu Benzophenonimin entstehen äquimolare Mengen an Benzophenonimin und Wasser. Eine Aufarbeitung dieses Reaktionsaustrags erfolgte bislang in der Regel nicht.

So ist in der US 4,130,586 ein Verfahren zur Herstellung von Benzophenoniminen beschrieben, wobei der erhaltene Reaktionsaustrag mit Benzol verdünnt wird. Eine weitere Aufarbeitung ist nicht beschrieben.

EP-A-0 713 861 betrifft ein Verfahren zur Herstellung von Benzophenoniminen durch Umsetzung von Benzophenonen in flüsigem Ammoniak in Gegenwart von oxidischen Katalysatoren. Insbesondere wird in Gegenwart von Titandioxid-Pulver als Katalysator umgesetzt. Aus dem erhaltenen Reaktionsäustrag wird Ammoniak abdestilliert, und der Katalysator wird abgetrennt. Ein so erhaltener zweiphasiger Austrag wird mit Dimethylformamid homogenisiert. Alternativ wird eine methanolische Benzophenonlösung in der Umsetzung eingesetzt, wobei wiederum Ammoniak abdestilliert wird und der Katalysator abgetrennt wird. Eine weitere Aufarbeitung des Produktes ist nicht beschrieben.

In G. Verardo, A.G. Giumanini, Synthetic Communications, 18(13), 1501 bis 1511 (1988) ist die Herstellung von Ketiminen aus Ketonen und Ammoniak beschrieben. Dabei wird Ammoniumchlorid als Katalysator eingesetzt. Zur Aufarbeitung wird das Reaktionsgemisch in Wasser und Ether gegossen, die organische Schicht abgetrennt und über Natriumsulfat getrocknet. Sodann wird eine Lösung von Chlorwasserstoff in trockenem Ether eingetragen und das ausgefällte Iminiumsalz gesammelt. Dieses Aufarbeitungsverfahren erfordert den Einsatz von organischen Lösungsmitteln, was es für einen technischen Einsatz tendenziell ungeeignet macht. Zudem wird das Iminiumsalz isoliert und nicht das Imin.

Ein technisch umsetzbares Aufarbeitungsverfahren für Benzophenonimin-Reaktionsausträge ist nicht bekannt.

Aus der Literatur ist zudem bekannt, daß Ketimine durch alkalische Lösungen zu Ketonen hydrolysiert werden.

Es wurde nun gefunden, daß bei der Lagerung eines wasserhaltigen Benzophenonimin-Reaktionsaustrags in erheblichem Maße eine Rückreaktion zu Benzophenon und Ammoniak stattfindet, die etwa 2 % pro Woche bei einer Lagerung bei 40°C betragen kann. Für eine ausreichende Lagerstabilität des Benzophenonimins sollte somit das im Reaktionsaustrag enthaltene Wasser abgetrennt werden.

Aufgabe der vorliegenden Erfindung ist die Bereitstellung eines Verfahrens zur Entfernung von Wasser und Ammoniak aus Benzophenonimin-Reaktionsausträgen, das in technischem Maßstab durchgeführt werden kann und bei dem keine Rückreaktion zu Benzophenon stattfindet.

Die Aufgabe wird gelöst durch ein Verfahren zur Entfernung von Wasser und Ammoniak aus Benzophenonimin-Reaktionsausträgen aus der katalytischen Umsetzung von Benzophenonen der allgemeinen Formel I 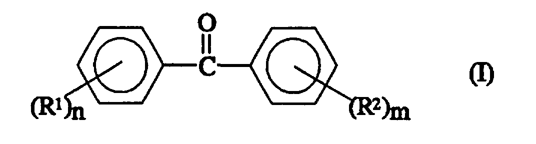 in der die Reste R¹ und R² unabhängig
a) Halogenatome, Hydroxylgruppen, Nitrogruppen, Aminogruppen,
b) geradkettige, verzweigte oder cyclische Alkylreste oder O-Alkylreste mit 1 bis 12 C-Atomen, die ihrerseits mit C₆₋₁₀-Aryl, F, Cl, Br substituiert sein können,
c) Arylreste oder O-Arylreste mit 6 bis 10 C-Atomen, die ihrerseits mit C₁₋₁₂-Alkyl oder -O-Alkyl substituiert sein können,
d) Heteroalkylreste, in denen die wie oben definierten Alkylreste, durch ein oder mehrere Heteroatome, ausgewählt aus O, S und N, unterbrochen sind,
e) Heteroarylreste mit 5 bis 10 Ringatomen, die 1 bis 3 Heteroatome, ausgewählt aus O, S und N, enthalten,
darstellen,
wobei m und n unabhängig ganze Zahlen von 0 bis 5 sind,
mit Ammoniak,
wobei eine Phasentrennung des Reaktionsaustrags in eine Ammoniak enthaltende Wasserphase und eine noch Wasser und Ammoniak enthaltende organische Phase durchgeführt wird und die Wasserphase entfernt wird
und sodann in einer Strippkolonne oder einem Dünnschichtverdampfer aus der organischen Phase das Ammoniak und das Wasser gleichzeitig abdestilliert werden,
oder sodann zuerst das Ammoniak aus der organischen Phase mit einem Fallfilmverdampfer abdestilliert wird und das Wasser anschließend mit einem Molekularsieb entfernt wird.

Es wurde dabei gefunden, daß die Entfernung von Ammoniak und Wasser aus Benzophenonimin-Reaktionsausträgen auf destillativem Wege möglich ist, ohne daß eine wie vorstehend beschriebene Rückreaktion des Benzophenonimins zu Benzophenon in nennenswertem Umfang auftritt. Insbesondere bei einer Reaktionsführung mit geringer thermischer Belastung kann die bei hoher Temperatur verstärkt auftretende Rückreaktion verhindert oder weitgehend unterdrückt werden.

Typische Reaktionsausträge bei der katalytischen Umsetzung von Benzophenonen mit Ammoniak zu Benzophenoniminen enthalten etwa 82% Benzophenonimin, etwa 4% Benzophenon, etwa 9% Wasser und etwa 5% Ammoniak. Dies ist insbesondere bei einer Synthese aus Benzophenon und flüssigem Ammoniak an TiO₂ als Katalysator der Fall.

Ein Teil des Wassers und des Ammoniaks bilden dabei eine Wasserphase aus, die sich von der organischen Phase trennt. Die Wasserphase enthält etwa 7 Gew.-% des bei der Reaktion entstehenden Wassers und etwa 2 Gew.-% des Ammoniaks, bezogen auf die Gesamtmenge des Reaktionsaustrags. Die verbleibenden etwa 2 Gew.-% Wasser und etwa 3 Gew.-% Ammoniak bleiben dabei in der organischen Phase aus Benzophenonimin und Benzophenon gelöst.

Es wird vor der Destillation eine Phasentrennung des Reaktionsaustrags in eine Ammoniak enthaltende Wasserphase und eine noch Wasser und Ammoniak enthaltende organische Phase durchgeführt und die Wasserphase entfernt. Das so erhaltene Gemisch, das im wesentlichen Benzophenonimin, Benzophenon, Ammoniak und Wasser enthält, wird sodann der Destillation zugeführt.

Die Destillation erfolgt dabei vorzugsweise bei einer Temperatur von 20 bis 200°C und einem Druck von 1 bis 1000 mbar.

Gemäß einer Ausführungsform der Erfindung werden Ammoniak und Wasser in der gleichen Destillationsapparatur gleichzeitig abdestilliert. Hierdurch wird das Aufarbeitungsverfahren vereinfacht und der apparative Aufwand gering gehalten. Die Destillation wird so durchgeführt, daß die thermische Belastung des Reaktionsaustrags möglichst gering ist, um eine Rückreaktion des Benzophenonimins zum Benzophenon zu verhindern. Insbesondere wird deshalb bei vermindertem Druck gearbeitet. Die Destillation erfolgt in einer Strippkolonne oder einem Dünnschichtverdampfer.

Gemäß einer Ausführungsform der Erfindung wird die Destillation mit einer Strippkolonne durchgeführt. Bei einer Kolonnentemperatur von vorzugsweise 20 bis 200°C, besonders bevorzugt 50 bis 100°C und einem Druck von vorzugsweise 100 bis 1000 mbar, besonders bevorzugt 200 bis 500 mbar werden Wasser und Ammoniak unter Strippen mit einem Inertgas, vorzugsweise Stickstoff, über Kopf entfernt. Das wasser- und ammoniakfreie Benzophenonimin wird dabei als Ablauf erhalten. Die Verweilzeit in der Destillationsblase liegt im Sekundenbereich. Eine Zersetzung des Benzophenonimins tritt nicht auf.

Gemäß einer besonders bevorzugten Ausführungsform der Erfindung wird die Destillation in einem Dünnschichtverdampfer ausgeführt. Dabei kann je nach Verdampfungsbedingungen das Benzophenonimin als Sumpfablauf oder als Destillat erhalten werden. Bei einer Ölvorlauftemperatur von vorzugsweise 80 bis 200°C, besonders bevorzugt 100 bis 160°C, insbesondere 100 bis 130°C und einem Druck von vorzugsweise 5 bis 200 mbar, besonders bevorzugt 20 bis 100 mbar fällt das Benzophenonimin als Sumpfablauf bzw. Hochsieder an. Die Verweilzeit im Dünnschichtverdampfer liegt hierbei im Sekundenbereich und ist vorzugsweise kürzer als bei der Strippkolonne. Bei einer Ölvorlauftemperatur von vorzugsweise 150 bis 200°C, besonders bevorzugt 160 bis 180°C und einem Druck von vorzugsweise 1 bis 20 mbar, besonders bevorzugt 5 bis 15 mbar wird das Benzophenonimin über Kopf abgezogen. Das Benzophenonimin wird dabei wasserfrei erhalten. Wasser und Ammoniak werden in beiden Fahrweisen über das Vakuumsystem entfernt. Die Verweilzeit im Dünnschichtverdampfer ist hierbei sehr kurz.

Gemäß einer weiteren Ausführungsform der Erfindung werden Ammoniak und Wasser nacheinander abgetrennt. Dabei wird zunächst Ammoniak und anschließend Wasser abgetrennt. Dabei erfolgt die Ammoniakabtrennung destillativ und die Wasserabtrennung nicht-destillativ mit einem Trockenmittel nach dem Abdestillieren von Ammoniak.

Dabei wird zuerst das Ammoniak mit einem Fallfilmverdampfer aus dem Reaktionsgemisch entfernt. Dabei beträgt die Öltemperatur des Verdampfers vorzugsweise 60 bis 200°C, besonders bevorzugt 80 bis 140°C, insbesondere 80 bis 120°C. Der Druck beträgt vorzugsweise 5 bis 200 mbar, besonders bevorzugt 20 bis 100 mbar, insbesondere 20 bis 60 mbar. Die Verweilzeit im Fallfilmverdampfer liegt dabei im Sekundenbereich.

Für die nicht-destillative Wasserentfernung sollten die Reaktionsausträge, insbesondere die Benzophenonimine vorzugsweise bei 25°C oder Raumtemperatur in flüssiger Form vorliegen. Bei höher schmelzenden Benzophenoniminen kann bei entsprechend höherer Temperatur gearbeitet werden, wobei jedoch die thermische Belastung möglichst gering gehalten werden soll.

Dabei wird der vom Ammoniak befreite Benzophenonimin-Reaktionsaustrag nicht-destillativ durch Behandlung mit Molekularsieb vom Wasser befreit.

Vorzugsweise wird Molekularsieb eingesetzt, beispielsweise vom Typ 514, 4 Å.

Gemäß einer Ausführungsform der Erfindung wird dabei der vom Ammoniak befreite Benzophenonimin-Reaktionsaustrag (Rohbenzophenonimin) kontinuierlich durch wechselseitigen Betrieb zweier Molekularsieb-Säulen vom Wasser befreit. Der Austrag wird kontinuierlich über eine erste mit Molekularsieb gefüllte Säule geleitet, wobei die Verweilzeit so gewählt wird, daß eine vollständige Entwässerung eintritt. Sobald ein Auftreten von Wasser im Benzophenoniminablauf aus der Säule festgestellt wird, d.h. die Kapazität des Molekularsiebes erschöpft ist, wird der Reaktionsaustrag über eine zweite Molekularsieb-Säule geleitet. Das mit Wasser beladene Molekularsieb der ersten Säule kann während dieser Zeit regeneriert werden, beispielsweise durch Erhitzen. Durch den wechselseitigen Betrieb der beiden Säulen kann dabei eine kontinuierliche Fahrweise ermöglicht werden.

Das erfindungsgemäße Verfahren zur Entfernung von Wasser und Ammoniak aus Benzophenonimin-Reaktionsausträgen ist für Benzophenonimin-Reaktionsausträge mit einem beliebigen Wasser- und Ammoniakgehalt geeignet. Es können dabei Reaktionsausträge aus allen bekannten Syntheseverfahren zur Herstellung von Benzophenoniminen durch katalytische Umsetzung von Benzophenonen mit Ammoniak eingesetzt werden.

Die erfindungsgemäß erhaltenen Benzophenonimine bzw. Benzophenonimin/Benzophenon-Gemische sind im wesentlichen frei von Ammoniak und Wasser. Vorzugsweise liegt der Wassergehalt der erhaltenen Benzophenonimine unterhalb von 0,07 Gew.-%, besonders bevorzugt unterhalb von 0,03 Gew.-%, bezogen auf das Gesamtgemisch. Das so erhaltene Benzophenonimin bzw. Benzophenonimin/Benzophenon-Gemisch ist dabei lagerstabil. Stabilitätsuntersuchungen des so erhaltenen, im wesentlichen wasserfreien Benzophenonimins bei Raumtemperatur zeigten keine Rückreaktion bzw. Zersetzung des Benzophenonimins im Rahmen der Analysengenauigkeit einer gaschromatographischen Analyse.

Das erhaltene, im wesentlichen von Wasser und Ammoniak freie Benzophenonimin bzw. Benzophenonimin/Benzophenon-Gemisch ist praktisch farblos. Die Farbzahl, gemessen nach Apha, liegt im allgemeinen im Bereich von 20 bis 50 Hazen. Die Messung erfolgt dabei nach DIN ISO 6271.

Vorzugsweise wird die thermische Belastung bei der destillativen Aufarbeitung der Benzophenonimin-Reaktionsausträge so gering gehalten, daß ein farbloses Benzophenonimin erhalten wird.

Erfindungsgemäß einsetzbare Benzophenonimin-Reaktionsausträge enthalten Benzophenonimine der allgemeinen Formel (II)

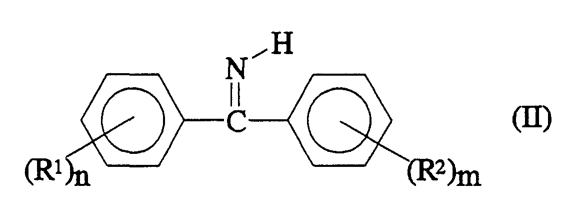

Diese werden hergestellt durch katalytische Umsetzung von Benzophenonen der allgemeinen Formel (I) 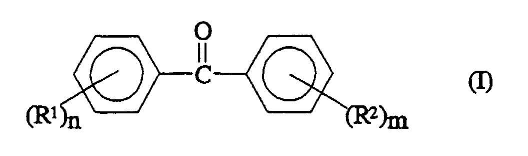 in der die Reste R¹ und R² unabhängig
a) Halogenatome, Hydroxylgruppen, Nitrogruppen, Aminogruppen,
b) geradkettige, verzweigte oder cyclische Alkylreste oder O-Alkylreste mit 1 bis 12 C-Atomen, die ihrerseits mit C₆₋₁₀-Aryl, F, Cl, Br substituiert sein können,
c) Arylreste oder O-Arylreste mit 6 bis 10 C-Atomen, die ihrerseits mit C₁₋₁₂-Alkyl oder -O-Alkyl substituiert sein können,
d) Heteroalkylreste, in denen die wie oben definierten Alkylreste, durch ein oder mehrere Heteroatome, ausgewählt aus O, S und N, unterbrochen sind,
e) Heteroarylreste mit 5 bis 10 Ringatomen, die 1 bis 3 Heteroatome, ausgewählt aus O, S und N, enthalten,
darstellen,
wobei m und n unabhängig ganze Zahlen von 0 bis 5 sind,
mit Ammoniak.

Vorzugsweise sind dabei die Reste R¹ und R² unabhängig voneinander Hydroxylgruppen, Nitrogruppen, geradkettige oder verzweigte Alkylreste oder O-Alkylreste mit 1 bis 4 C-Atomen, Phenylreste oder O-Phenylreste, die gegebenenfalls mit C₁₋₄-Alkyl oder C₁₋₄-O-Alkyl substituiert sind.

Vorzugsweise sind m und n unabhängig ganze Zahlen von 0 bis 2, besonders bevorzugt 0 oder 1.

Insbesondere ist m = n = 0, d.h. Benzophenon wird zur Umsetzung mit Ammoniak zu Benzophenonimin eingesetzt.

Die Erfindung wird nachstehend anhand von Beispielen erläutert.

In den Beispielen wurde ein Benzophenonimin-Reaktionsaustrag verwendet, der nach dem nachstehenden Verfahren erhalten wurde.

Durch einen Rohrreaktor, der mit 60 ml Titandioxid im Form von 3 mm-Strängen gefüllt war, wurden 180 g Benzophenon und 720 g Ammoniak pro Stunde bei einem Druck von 200 bar und einer Temperatur von 130°C gefahren. Vom Reaktionsaustrag wurde sodann die Wasserphase abgetrennt und die organische Phase in den nachfolgenden Beispielen eingesetzt. Sie bestand aus 89,8 Gew.-% Benzophenonimin, 5,28 Gew.-% Benzophenon, 1,82 Gew.-% Wasser und 3,10 Gew.-% Ammoniak.

### BEISPIEL 1

500 g des Benzophenonimin-Austrags wurden kontinuierlich auf einen Dünnschichtverdampfer bei einer Öltemperatur von 100 bis 120°C und einem Druck von 9 mbar gepumpt. Es wurden dabei insgesamt 441 g wasserfreies, farbloses Benzophenonimingemisch mit einem Gehalt von 94,3 Gew.-% Benzophenonimin und 5,68 Gew.-% Benzophenon als Sumpfablauf erhalten. Das Produkt wies eine Farbzahl von 35 Hazen, gemessen nach Apha, auf. Der Wassergehalt betrug 0,03 Gew.-%, bestimmt nach Karl-Fischer, vgl. DIN 51777, Teil 1, direktes Verfahren.

### BEISPIEL 2

500 g des Benzophenonimin-Austrags wurden kontinuierlich über einen Dünnschichtverdampfer mit einer Öltemperatur von 160 bis 180°C bei einem Druck von weniger als 10 mbar destilliert. Es wurden insgesamt 422 g wasserfreies, farbloses Benzophenonimingemisch mit einem Benzophenonimingehalt von 94,3 Gew.-% und einem Benzophenongehalt von 5,56 Gew.-% als Kopfprodukt erhalten. Das Produkt wies eine Farbzahl von 28 Hazen, gemessen nach Apha, auf. Der Wassergehalt betrug 0,02 Gew.-%, bestimmt nach Karl-Fischer.

### BEISPIEL 3

250 g des Benzophenonimin-Austrags wurde zur Entfernung des Ammoniaks kontinuierlich auf einen Fallfilmverdampfer mit einer Öltemperatur von 100°C bei einem Druck von 40 mbar gepumpt. Es wurden dabei 228 g ammoniakfreies Roh-Benzophenonimin als Sumpfablauf erhalten. Dieses Produkt mit einem Wassergehalt von etwa 1,6 Gew.-% wurde mit 47 g Molekularsieb (Typ 514, 4 Å) versetzt und bei Raumtemperatur gerührt. Nach 4 Stunden wurde das Molekularsieb abfiltriert, wobei 214 g farbloses Benzophenonimingemisch mit einem Gehalt an Benzophenonimin von 94,1 Gew.-% und Benzophenon von 5,79 Gew.-% erhalten wurden. Die Farbzahl betrug 22 Hazen, gemessen nach Apha, und der Wassergehalt betrug 0,6 Gew.-%, bestimmt nach Karl-Fischer.

Das Verhältnis von Benzophenon zu Benzophenonimin war in den Beispielen 1 und 2 im Produkt gleich dem Verhältnis im eingesetzten Reaktionsaustrag, in Beispiel 3 geringfügig höher. Dies zeigt, daß bei der Aufarbeitung keine oder nur sehr geringe Rückreaktion bzw. Zersetzung des Benzophenonimins stattfindet. Das erfindungsgemäße Verfahren ist somit vorteilhaft zur Reinigung von Benzophenonimin-Reaktionsausträgen einsetzbar, um ein lagerfähiges Produkt zu erhalten.

Benzophenonimin wird technisch als Ausgangsverbindung für Lichtschutzmittel eingesetzt.

## Patentansprüche

1. Verfahren zur Entfernung von Wasser und Ammoniak aus Benzophenonimin-Reaktionsausträgen aus der katalytischen Umsetzung von Benzophenonen der allgemeinen Formel I 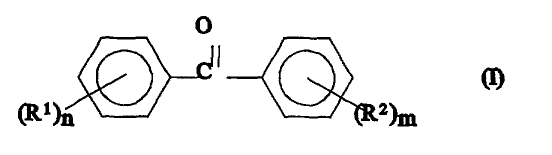 in der die Reste R¹ und R² unabhängig
a) Halogenatome, Hydroxylgruppen, Nitrogruppen, Aminogruppen,
b) geradkettige, verzweigte oder cyclische Alkylreste oder O-Alkylreste mit 1 bis 12 C-Atomen, die ihrerseits mit C₆₋₁₀-Aryl, F, Cl, Br substituiert sein können,
c) Arylreste oder O-Arylreste mit 6 bis 10 C-Atomen, die ihrerseits mit C₁₋₁₂-Alkyl oder -O-Alkyl substituiert sein können,
d) Heteroalkylreste, in denen die wie oben definierten Alkylreste, durch ein oder mehrere Heteroatome, ausgewählt aus O, S und N, unterbrochen sind,
e) Heteroarylreste mit 5 bis 10 Ringatomen, die 1 bis 3 Heteroatome, ausgewählt aus O, S und N, enthalten,
darstellen,
wobei m und n unabhängig ganze Zahlen von 0 bis 5 sind,
mit Ammoniak,
wobei eine Phasentrennung des Reaktionsaustrags in eine Ammoniak enthaltende Wasserphase und eine noch Wasser und Ammoniak enthaltende organische Phase durchgeführt wird und die Wasserphase entfernt wird
und sodann in einer Strippkolonne oder einem Dünnschichtverdampfer aus der organischen Phase das Ammoniak und das Wasser gleichzeitig abdestilliert werden,
oder sodann zuerst das Ammoniak aus der organischen Phase mit einem Fallfilmverdampfer abdestilliert wird und das Wasser anschließend mit einem Molekularsieb entfernt wird.

## Claims

1. A process for removing water and ammonia from benzophenone imine reactor effluents resulting from the catalytic reaction of benzophenones of the formula I 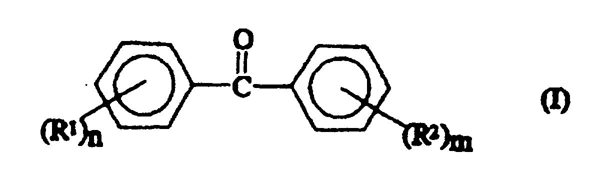 where R¹ and R², independently, are
a) halogen, hydroxyl, nitro, amino,
b) straight-chain, branched or cyclic alkyl or O-alkyl having from 1 to 12 carbon atoms, which may themselves be substituted with C₆₋₁₀-aryl, F, Cl, Br,
c) aryl or O-aryl having from 6 to 10 carbon atoms, which may themselves be substituted with C₁₋₁₂-alkyl or -O-alkyl,
d) heteroalkyl in which alkyl radicals defined as above are interrupted by one or more heteroatoms selected from the class consisting of O, S and N,
e) hetaryl having from 5 to 10 ring atoms including from 1 to 3 heteroatoms selected from the class consisting of O, S and N,
where m and n, independently, are integers from 0 to 5,
with ammonia,
where the reactor effluent is separated by phase into an ammonia-containing aqueous phase and an organic phase in which water and ammonia remain present, and the aqueous phase is removed
and then the ammonia and the water are simultaneously distilled off from the organic phase in a stripping column or in a film evaporator,
or then first the ammonia is distilled off from the organic phase, using a falling-film evaporator, and the water is then removed, using a molecular sieve.

## Revendications

1. Procédé d'élimination d'eau et d'ammoniac à partir d'effluents réactionnels de la préparation de benzophénone-nimine provenant de la réaction catalytique de benzophénone de la formule générale I: 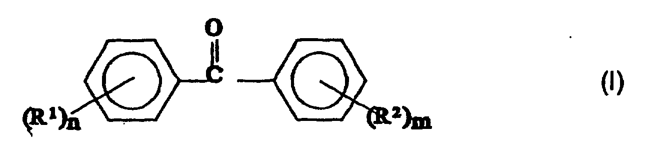 dans laquelle les radicaux R¹ et R² représentent indépendamment
a) des atomes d'halogène, des groupes hydroxyle, des groupes nitro, des groupes amino,
b) des radicaux alkyle ou O-alkyle linéaires, ramifiés ou cycliques, comportant 1 à 12 atomes de C, qui à leur tour peuvent être substitués par de l'aryle en C₆-C₁₀, F, Cl, Br,
c) des radicaux aryle ou 0-aryle comportant 6 à 10 atomes de C, qui à leur tour peuvent être substitués par un alkyle ou O-alkyle en C₁-C₁₂,
d) des radicaux hétéroalkyliques, dans lesquels les radicaux alkyle définis précédemment sont interrompus par un ou plusieurs hétéroatomes, choisis parmi O, S et N,
e) des radicaux hétéroaryle comportant 5 à 10 atomes dans le noyau et contenant 1 à 3 hétéroatomes choisis parmi O, S et N,
m et n représentant indépendamment des nombres entiers de 0 à 5,
avec de l'ammoniac,
dans lequel on effectue une séparation de phases dans l'effluent réactionnel en une phase aqueuse contenant l'ammoniac et une phase organique contenant encore de l'eau et de l'ammoniac, et on élimine la phase aqueuse,
et ensuite, dans une colonne de stripage ou un évaporateur en couche mince, on sépare par distillation de la phase organique simultanément l'ammoniac et l'eau,
ou ensuite on sépare tout d'abord l'ammoniac par distillation de la phase organique à l'aide d'un évaporateur pelliculaire et ensuite on élimine l'eau à l'aide d'un tamis moléculaire.
